# EUROPÄISCHE PATENTANMELDUNG

(11) **EP 0 693 685 A1**
(43) Veröffentlichungstag der Anmeldung: **24.01.1996**
(21) Anmeldenummer: 94111440.7
(22) Anmeldetag: 22.07.1994
(51) Int. Cl.: G01N 31/16, G01N 35/04

(54) **Vorrichtung zum Bestimmen des Stickstoff- und Proteingehalts, insbesondere von Lebensmitteln**

(71) Anmelder: RESONA INNOVATION AG, CH-9202 Gossau (CH)
(72) Erfinder: Zellweger, Adolf, CH-9202 Gossau (CH)
(74) Vertreter: Göbel, Matthias, Dipl.-Ing.

(57) **Zusammenfassung**

Bei einer Vorrichtung zum Bestimmen des Stickstoff- und Proteingehalts, insbesondere von Lebensmitteln, nach der Kjeldahl-Methode mit einander folgenden Prozeßstationen für die Vorbereitung mit Einwägung, Aufschließung, Kühlung, Destillation und Tritation von in Aufnahmegefäßen untergebrachten Proben, sind zur sicheren und übersichtlichen Handhabung der Vorrichtung, die Proben in zu Kassetten (3) gefaßten Aufnahmegefäßen (2) eingebracht und die Kassetten auf einer Schienenbahn (21) od.dgl. manuell oder motorisch in die Prozeßstationen (1, 4, 7, 8, 15) für die Vorbereitung mit Einwägung, Aufschließung, Kühlung, Destillation und Tritation verfahr- und/oder verschiebbar.

## Beschreibung

Die Erfindung betrifft eine Vorrichtung zum Bestimmen des Stickstoff- und Proteingehalts, insbesondere von Lebensmitteln, nach der Kjeldahlmethode, mit einander folgenden Prozeßstationen für die Vorbereitung mit Einwägung, Aufschließung, Kühlung, Destillation und Tritation von in Aufnahmegefäßen untergebrachten Proben.

Bei Vorrichtungen dieser Art ist es bekannt, die Prozeßstationen getrennt einzeln auszubilden und die in gläsernen Aufnahmegefäßen befindlichen Proben in komplizierter Weise, z.B. mittels Werkzeugen, nacheinander in die einzelnen Prozeßstationen einzustellen. Diese Handhabung der Proben hat sich als unsicher und unübersichtlich gezeigt und macht zusätzliche Kontrollvorgänge erforderlich.

Es ist Aufgabe der Erfindung Maßnahmen zur sicheren und übersichtlichen Handhabung einer Vorrichtung obiger Gattung zu schaffen.

Gemäß der Erfindung ist diese Aufgabe dadurch gelöst, daß die Proben in zu Kassetten gefaßten Aufnahmegefäßen eingebracht und die Kassetten auf einer Schienenbahn od.dgl. manuell oder motorisch in die Prozeßstationen für die Vorbereitung mit Einwägung, Aufschließung, Kühlung, Destillation und Tritation verfahrbar- und/oder verschiebbar sind. Auf diese Weise ist eine Fahrstraße für die Proben gebildet, auf der die Kassetten mit den die Proben aufnehmenden Aufnahmegefäßen in die Prozeßstationen zeitsparend und sicher sowie übersichtlich einfach verbringbar sind, wobei das Erkennen der Proben in den einzelnen Prozeßstationen gewährleistet ist. Zweckmäßig sind die Kassetten auf der Schienenbahn vermittels Rollen od. dgl. leichtgängig verfahrbar. Die Schienenbahn kann dabei durch mindestens eine Schiene, bevorzugt zwei Schienen gebildet sein, wobei die Schienenbahn die Prozeßstationen zweckmäßig miteinander verbindet.

Nach bevorzugter Ausbildung der Vorrichtung kann sich die Schienenbahn sowohl über Prozeßstationen für die Vorbereitung mit Einwägung, Aufschließung, Kühlung, Destillation, Tritation der Proben als auch über eine Prozeßstation für den Probenabbau durch Recycling erstrecken.

In Ausgestaltung der Vorrichtung können bevorzugt die Prozeßstationen Einzelmodule bilden, die linear aneinander reihbar und justierbar sind. Darüberhinaus besteht in Abwandlung der Vorrichtung die Möglichkeit, die modularen Prozeßstationen auf bögenförmigen Flächen, insbesondere kreisringförmigen Flächen nebeneinander anzuordnen und zu justieren. Es versteht sich, daß die Prozeßstationen gemeinsam auch eine Einheit bilden können, wobei die modularen Prozeßstationen oder die durch Einheiten gebildeten Kjeldahl-Systeme im wesentlichen mit gleicher Höhe ausgebildet sind und einen insbesondere im wesentlichen prismatischen oder zylindrischen Umriß aufweisen.

Die Prozeßstation für die Aufschließung der Proben kann in Ausbildung der Vorrichtung einen Heizblock und einen Hebelift sowie eine, insbesondere schwenkbare Gasabsaugvorrichtung mit Verbindungschläuchen zum Abzug der Gase aus den Aufnahmegefäßen der Proben zu einer Gasreinigungseinheit aufweisen.

Die Prozeßstation für die Kühlung ist nach weiterer Ausbildung der Vorrichtung durch einen Kasten oder ein kastenförmiges Gehäuse gebildet und weist in oder an einer Seitenwand, z.B. der Rückwand, ein Kühlgebläse oder eine Kühlluftzuführung auf die z.B. über eine Kühlluftschlange in die Prozeßstation für die Kühlung einmündet.

Fernerhin ist vorgesehen, die Prozeßstation für die Destillation mit einem waag- und senkrecht bewegbaren Kopfsystem mit Spritzschutz, Kühler und Dosierrohr zum Anfahren der in den Kassetten befindlichen Aufnahmegefäßen für die Proben zu versehen. Darüberhinaus kann die Prozeßstation für die Destillation mindestens einen Unter- oder Anbau mit Vorratsbehälter für die Aufnahme von Wasser und Chemikalien, z.B. Natriumhydroxyd (NaOH) aufweisen.

Schließlich ist in weiterer Ausgestaltung der Vorrichtung vorgesehen, die Prozeßstationen manuell oder durch Kontrolleinheiten selbständig anzusteuern und den Programmablauf in den Prozeßstationen, z.B. der Temperatur, und Reinigung der Gase sowie Betätigung eines Hebelifts und Heizblocks individuell oder programmierbar ablaufen zu lassen.

Von Vorteil hat sich außerdem gezeigt, wenn die Prozeßstationen, die Kontroll- und Steuereinheiten für diese sowie die Schienenbahn aus einem nichtrostenden, bevorzugt mit gebürsteter Oberfläche, oder einem laugenbeständigen Werkstoff gebildet sind. Hierdurch ist eine störfreie Benutzung der Vorrichtung über einen längeren Zeitraum gewährleistet. Außerdem können der Prozeßstation für den Probenabbau durch Recycling noch zusätzliche Abbaumodule zugeordnet sein, um so die Aufnahmekapazität dieser Prozeßstation zu erweiteren.

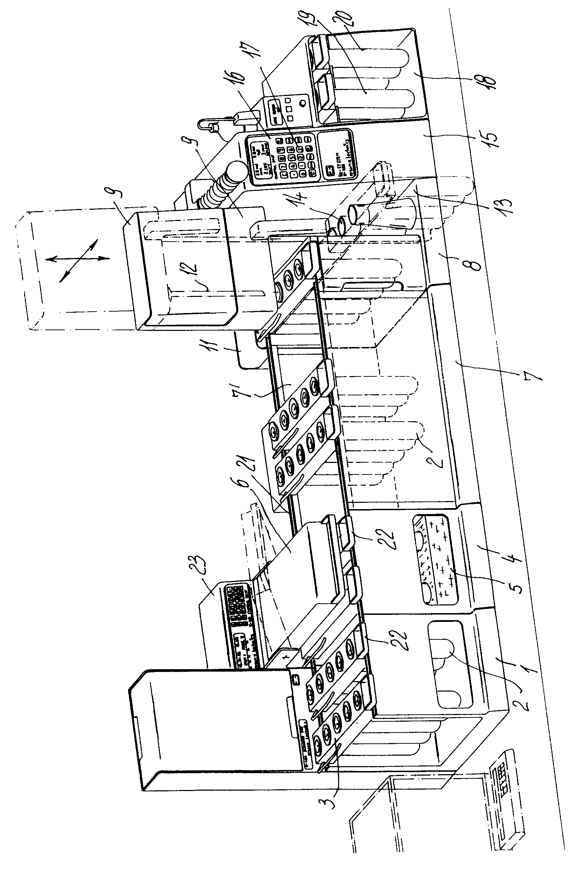

Die Erfindung ist anhand eines in der Zeichnung dargestellten Ausführungsbeispiels erläutert.

Die nach der Kjeldahl-Methode arbeitende Vorrichtung ist als Prozeßstraße mit einander folgenden Prozeßstationen ausgeführt. Beim Ausführungsbeispiel sind die Prozeßstationen durch aneinander gereihte und justierte Einzelmodule gebildet.

Mit 1 ist eine Prozeßstation für die Einwägung und Vorbereitung der Proben bezeichnet. Die Proben, z.B. feste oder flüssige Lebensmittel sind in Aufnahmegefäßen 2, z.B. aus Glas, eingefüllt, wobei jeweils mehrere Aufnahmegefäße 2 zu Kassetten 3 zusammengefaßt sind. Der Prozeßstation 1 folgt eine Prozeßstation 4, die der Aufschließung und der Gasreinigung der aus den Aufnahmegefäßen 2 für die Proben abgezogenen Gase dient. Die Prozeßstation 4 ist mit einem Heizblock 5 und einer schwenkbaren Absaugvorrichtung 6 mit Verbindungsschläuchen zur Gaswascheinheit und einem Hebelift versehen. Außerdem ist eine Kontrolleinheit 23 zugeordnet. Mit 7 ist eine Prozeßstation für die Kühlung der Proben bezeichnet, die beim Ausführungsbeispiel kastenförmig ausgebildet ist und in bzw. an der Rückwand 7' einen Ventilator (nicht gezeigt) zur Lieferung der Kühlluft aufweist. Es versteht sich, daß Kühlluft auch anderweitig, z.B. über ein Schlauchsystem von einem außerhalb der Prozeßstation 7 aufgestellten Kühlgebläse in die Prozeßstation lieferbar ist. Die folgende Prozeßstation 8 für die Destillation weist ein waag- und senkrecht bewegbares Kopfsystem 9 mit Spritzschutz 10, Kühler 11 und Dosierrohr 12 zum Anfahren der in den Kassetten 3 befindlichen Aufnahmegefäßen 2 auf. Weiter ist der Prozeßstation 8 ein Unter- oder Anbau 13 mit Behältern 14 für die Aufnahme von Wasser und Chemikalien, z.B. Natriumhydroxyd zugeordnet. Mit der Prozeßstation 8 steht eine Prozeßstation 15 für Tritation in Verbindung, die an einem Computer 16 mit Anzeigen 17 anliegt. 18 ist eine Prozeßstation mit Aufnahmen 19, für die Probenablage zum Abbau derselben im Recyclingverfahren. Die Kapazität der Prozeßstation 18 kann durch weitere Ablagemodule 20 ausgebaut werden.

Beim Ausführungsbeispiel ist mit 21 eine zweispurige Schienenbahn bezeichnet, die sich über die Prozeßstationen 1, 4, 7, 8, 15 erstreckt. Auf der Schienenbahn 21 sind die Kassetten 3 vermittels kassettenfesten Bügeln 22 abgestützt und verfahr- oder verschiebbar. Die Fahr- und Verschiebebewegungen der Kassetten auf der Schienenbahn 21 lassen sich erleichtern durch die Anordnung von Handgriffen 22 an den Kassetten 3.

Während beim gezeigten Ausführungsbeispiel die Schienenbahn 21 in der Prozeßstation 8 für die Destillation endet, besteht in Abwandlung der Vorrichtung die Möglichkeit, die Schienenbahn 21 bis über die Prozeßstationen 15 und 18 für Tritation und für den Probenabbau hinwegzuführen. Die Prozeßstationen 1, 4, 7, 8, 15 und 18, die Kontroll- und Steuereinheiten 16, 23 für diese sowie die Schienenbahn 21 sind aus einem nichtrostenden und/oder laugenbeständigen Werkstoff, insbesondere mit gebürsteten Oberflächenstrukturen gebildet.

Fernhin können anstelle der zur Anwendung gebrachten Einzelmodule 1, 4, 7, 8, 15 und 18 die Kjeldahl-Systeme auch als bauliche Einheiten mit beliebigen, z.B. prismatischen oder zylindrischen Umrissen ausgeführt sein.

## Patentansprüche

1. Vorrichtung zum Bestimmen des Stickstoff- und Proteingehalts, insbesondere von Lebensmitteln, nach der Kjeldahl-Methode mit einander folgenden Prozeßstationen für die Vorbereitung mit Einwägung, Aufschließung, Kühlung, Destillation und Tritation von in Aufnahmegefäßen untergebrachten Proben, dadurch gekennzeichnet, daß die Proben in zu Kassetten (3) gefaßten Aufnahmegefäßen (2) eingebracht und die Kassetten auf einer Schienenbahn (21) od. dgl. manuell oder motorisch in die Prozeßstationen (1, 4, 7, 8, 15) für die Vorbereitung mit Einwägung, Aufschließung, Kühlung, Destillation und Tritation verfahr- und/ oder verschiebbar sind.

2. Vorrichtung nach Anspruch 1, dadurch gekennzeichnet, daß sich die Schienenbahn (21) od. dgl. über die Prozeßstationen (1, 4, 7, 8, 15), für die Vorbereitung mit Einwägung, Aufschließung, Kühlung, Destillation, Tritation der Proben und über eine Prozeßstation (18) für den Probenabbau durch Recycling erstreckt.

3. Vorrichtung nach Anspruch 1, dadurch gekennzeichnet, daß die die Aufnahmegefäße (2) für die Proben fassenden Kassetten (3) vermittels Rollen od. dgl. auf der Schienenbahn (21) verfahrbar sind.

4. Vorrichtung nach Anspruch 1 und 2, dadurch gekennzeichnet, daß die Prozeßstationen (1, 4, 7, 8, 15, 18) Einzelmodule bilden die linear aneinanderreih- und justierbar sind.

5. Vorrichtung nach Anspruch 1 und 2, dadurch gekennzeichnet, daß die modularen Prozeßstationen (1, 4, 7, 8, 15, 18) gemeinsam auf einer bogenförmigen Fläche, insbesondere kreisringförmigen Fläche nebeneinander angeordnet und justierbar sind.

6. Vorrichtung nach Anspruch 1 und 2, dadurch gekennzeichnet, daß Prozeßstationen (1, 4, 7, 8, 15, 18) gemeinsam eine prismatische bauliche Einheit bilden.

7. Vorrichtung nach Anspruch 5, dadurch gekennzeichnet, daß die Prozeßstationen (1, 4, 7, 8, 15, 18) gemeinsam eine bogenförmige bauliche Einheit bilden.

8. Vorrichtung nach Anspruch 4, 5 und 7, dadurch gekennzeichnet, daß die Prozeßstationen (1, 4, 7, 8, 15, 18) im wesentlichen mit gleicher Höhe ausgebildet sind und gemeinsam ein Kjeldahl-System mit im wesentlichen prismatischen oder zylindrischen Umriß bilden.

9. Vorrichtung nach Anspruch 1 und 2, dadurch gekennzeichnet, daß die Schienenbahn (21) durch mindestens eine Schiene, insbesondere zwei Schienen gebildet ist und daß die Schiene bzw. Schienen die Prozeßstationen (1, 4, 7, 8, 15, bzw. 1, 4, 7, 8, 15, 18) miteinander verbindet bzw. verbinden.

10. Vorrichtung nach Anspruch 1 und 2, dadurch gekennzeichnet, daß die Prozeßstationen (1, 4, 7, 8, 15, 18) manuell oder durch Kontrolleinheiten (16, 23) selbständig ansteuerbar und der Programmablauf in den Prozeßstationen individuell oder programmierbar abläuft.

11. Vorrichtung nach Anspruch 1 und 2, dadurch gekennzeichnet, daß die Prozeßstation (4) für die Aufschließung einen Heizblock (5) und einen Hebelift sowie eine insbesondere schwenkbare Gasabsaugvorrichtung mit Verbindungsschläuchen zu einer Gasreinigungseinheit aufweist.

12. Vorrichtung nach Anspruch 1 und 2, dadurch gekennzeichnet, daß die Prozeßstation (7) für die Kühlung durch einen Kasten oder ein kastenförmiges Gehäuse gebildet ist und in oder an einer Seitenwand, z.B. der Rückwand (7'), ein Kühlgebläse oder eine Kühlluftzuführung aufweist, die über eine Kühlschlange od.dgl. in die Prozeßstation (7) einmündet. .

13. Vorrichtung nach Anspruch 1 und 2, dadurch gekennzeichnet, daß die Prozeßstation (8) für Destillation ein waag- und senkrecht bewegbares Kopfsystem (9) mit Spritzschutz (10) Kühler (11) und Dosierrohr (12) zum Anfahren der in den Kasetten (3) befindlichen Aufnahmegefäßen (2) aufweist.

14. Vorrichtung nach Anspruch 13, dadurch gekennzeichnet, daß der Prozeßstation (8) für Destillation mindestens ein Unter- oder Anbau mit Aufnahmebehälter (14) für Wasser und Chemikalien, z.B. Natriumhydroxyd (NaOH) zugeordnet ist.

15. Vorrichtung nach Anspruch 1 und 2, dadurch gekennzeichnet, daß die Prozeßstationen ( 1, 4, 7, 8, 15, 18) die Kontroll- und Steuereinheiten (23) für diese sowie die Schienenbahn (21) aus einem nichtrostenden oder laugenbeständigen Werkstoff gebildet sind.

16. Vorrichtung nach Anspruch 2, dadurch gekennzeichnet, daß die Prozeßstation (18) für den Probenabbau durch Recycling vermittels zusätzlichen Abbaumodulen (20) in der Aufnahmekapazität erweiterbar ist.
